(19) Europäisches Patentamt

European Patent Office

Office européen des brevets

(11) EP 0 482 811 B1

## (12) EUROPEAN PATENT SPECIFICATION

(45) Date of publication and mention
of the grant of the patent:
**16.04.1997 Bulletin 1997/16**

(51) Int. Cl.⁶: **C01B 11/08**, A01N 25/22,
A61L 9/01

(21) Application number: **91309468.6**

(22) Date of filing: **15.10.1991**

(54) **Chlorous acid solutions**

Chlorigsäurelösungen

Solutions d'acide chloreux

(84) Designated Contracting States:
**BE DE ES FR GB IT NL**

(30) Priority: **26.10.1990 GB 9023387**

(43) Date of publication of application:
**29.04.1992 Bulletin 1992/18**

(73) Proprietor: **DIVERSEY LIMITED**
**Northampton NN3 8PD (GB)**

(72) Inventor: **Neil, Thomas Bruce**
**Middleton, Manchester M24 4DP (GB)**

(74) Representative: **Ablewhite, Alan James et al**
**MARKS & CLERK,**
**57/60 Lincoln's Inn Fields**
**London WC2A 3LS (GB)**

(56) References cited:
**DE-B- 1 007 745          FR-A- 959 544**
**US-A- 2 947 700**

• **PATENT ABSTRACTS OF JAPAN vol. 14, no. 275**
**(C-728)14 June 1990 & JP-A-2084 128**
**(HODOGAYA CHEM. CO.) 26 March 1990**

## Description

This invention relates to aqueous solutions of chlorous acid.

It has long been known that solutions of chlorous acid can bleach, deodorise and disinfect. The use of these solutions for the treatment of potable water and the bleaching of fabrics has been known for many years. More recently these solutions have been used for the disinfection of fruit, vegetables, salads and food preparation surfaces. In all of these applications the chlorous acid is obtained via the acidification of a sodium chlorite solution, the concentration of which determines the yield of chlorous acid.

Existing methods for activating sodium chlorite solutions to give chlorous acid include acidifying to a given pH in the range between pH 2 and pH 4, or mixing sodium chlorite and acid in stoichiometric proportions. The method that is employed depends upon the recommendations of the supplier. Neither of these approaches is desirable nor do they make the optimum use of the available chlorous acid.

Chlorous acid tends to decompose to chlorine dioxide and chloric acid. Chlorine dioxide gas is explosive and it is necessary to avoid the release of significant amounts of free chlorine dioxide gas. US 3082146 seeks to minimise losses to chlorine dioxide and chlorate by the addition of a peroxide, but still requires activation by addition of acid. Also in FR 959, 544 there is proposed a method of reducing chlorine dioxide formation in solution by the addition of phosphates and hydrogen peroxide.

For the disinfection of food preparation surfaces the F.D.A. (USA Food and Drugs Administration) have approved the use of chlorine dioxide solutions containing 100 to 200 ppm of "available chlorine dioxide". These "chlorine dioxide solutions" are effectively solutions of chlorous acid. Addition of a stoichiometric quantity of an acid to a sodium chlorite solution of concentration sufficient to yield chlorous acid solution equivalent to 100 to 200 ppm of chlorine dioxide results in the evolution of chlorine dioxide into the atmosphere. In the head space of sealed bottles in which the ratio of the head space: solution volumes were respectively 400 cm$^3$:100 cm$^3$, levels of chlorine dioxide well above the T.L.V. (Threshold Limit Value) of 0.1 ppm were found. The generation of chlorine dioxide under these conditions is potentially hazardous. The addition of sufficient acid to bring the pH within the range from pH 2.0 to 2.5 was found to release all of the available chlorous acid immediately and to cause a fairly rapid build up of chlorine dioxide gas above the solution. In all of the instances described here, the activated solutions rapidly developed a yellow colouration within a matter of minutes due to the high concentration of chlorine dioxide in the solution. The chlorine dioxide solutions produced as described above were found to lose their activity progressively with time.

Acidification to pH 4 is recommended by a major North American producer of chlorite solutions. The benefit of this approach is that liberation of chlorine dioxide gas could not be detected from sodium chlorite solutions containing the equivalent of 100 to 200 ppm available chlorine dioxide. However, this approach suffers from the disadvantage that at pH 4 there is only sufficient acid available to cause partial activation of the chlorite present. Typically, chlorous acid equivalent to 6 to 20 ppm of chlorine dioxide will be released from such a solution. Hence this approach does not convert all of the sodium chlorite present into chlorous acid.

The present invention has been made from a consideration of this.

According to a first aspect of the present invention there is provided an aqueous solution comprising a chlorite of an alkali metal or an alkaline earth metal and a buffer adapted to maintain the pH of the fluid substantially in the range from 3.5 to 6.0 and which has sufficient acid reserve to convert substantially all the chlorite to chlorous acid.

The pH may for example be in the range 3.5 to 5.0.

The buffered materials release chlorous acid less rapidly than materials which have been acidified without buffering. However the chlorous acid releasing solution is usually produced by the addition of liquid concentrates to water. Concentrated mixtures of chlorite and buffer tend to release high concentrations of chlorine dioxide into the atmosphere. Therefore the two separate concentrates of buffer solution and chlorite solution are mixed at the point of use via controlled dosing equipment. Alternatively the solution can be provided as a ready mixed dilute solution which has a substantially longer shelf life than a mixture of the concentrates.

According to a second aspect of the present invention there is provided a method of producing a chlorous acid releasing solution comprising mixing a chlorite of an alkali metal and/or an alkaline earth metal with a buffer adapted to maintain the pH of the mixture substantially in the range from 3.5 to 6.0.

The aqueous solutions of the present invention have valuable disinfectant and deodorizing properties.

In a preferred embodiment of the invention the fluid is a dilute aqueous (active) solution comprising 25 to 300 ppm available chlorous acid, more preferably 50 to 250 ppm.

The concentration of alkali metal chlorite, such as sodium chlorite, in the fluid is in the range from 20 to 350 and more preferably of the order of 180 ppm. In such a solution the concentration of chlorous acid is maintained at a very low but constant level. Sufficient buffer is provided to overcome the alkalinity of both the chlorite solution and of the water used which may arise from the presence of hardness salts in the water. There must also be sufficient residual acid (acid reserve) to convert substantially all of the available chlorite to chlorous acid. Preferred buffers comprise weak organic acids such as at least one of acetic acid and optionally a mineral acid such as phosphoric acid. The buffer also comprises a strong base, for example the hydroxide of an alkali metal such as sodium hydroxide. The amount of buffer in the dilute fluid may be in the range from 0.1 to 4.0% by weight with up to 360 ppm sodium chlorite present and more preferably in the order of 1% by weight.

The fluid in accordance with the present invention provides a means of generating chlorous acid in a gradual and controlled manner with consequent reduced risk in reaching the T.L.V. (Threshold Limit Value) of free chlorine dioxide gas.

The sodium chlorite solution is alkaline as it comprises the salt of a weak acid and a strong base and therefore undergoes hydrolysis in water. As stated above the buffer composition must provide sufficient acid to overcome the alkalinity of both the chlorite solution and of the water used. Also once the pH of the solution has been reduced to the desired pH sufficient residual acid is present to convert substantially all of the available chlorite to chlorous acid.

In order that the invention may be more readily understood specific embodiments thereof will now be described by way of example only.

A weak organic acid was selected for use in a buffer for use in a fluid in accordance with the invention. Acetic acid was selected as being suitable for commercial, toxicological and stability reasons. Buffer solutions were prepared comprising 1.8 to 2.0% sodium hydroxide, 10 to 15% acetic acid and from 3.0 to 0% phosphoric acid, depending upon the pH required.

It has been found that large amounts of acetic acid may be required in order to reduce the pH to the required acidity. In these circumstances it may be preferably to use a mineral acid such as phosphoric acid in combination with the acetic acid. This reduces the amount of acetic acid required to bring about the required acidity.

In use the preferred concentration of the buffer will be substantially 1% wt/wt. At this rate of use there is sufficient free acid to reduce all of the available chlorous acid. However, the buffer will continue to buffer the pH down to dilutions of 0.1 to 0.15% with up to 360 ppm of sodium chlorite, though the preferred concentration is in the order of 180 ppm. At these low dilutions only partial conversion of chlorite to chlorous acid occurs. Concentrations greater than 1.0% may be needed but should be used in equal parts with the chlorite solution.

It has been found that enhanced bactericidal effects are obtained if the chlorous acid releasing solution also contains a surfactant. The surfactant must of course be resistant to oxidation by the chlorous acid.

Examples of suitable surfactants are those based on amine oxides and ether sulphates based on 2 to 3 moles of ethylene oxide per molecule. The quantity of surfactant may for example correspond to the range 0.01 to 1% by weight in the diluted solution.

Rate of Release of Chlorous Acid (pH 3.5)

Example 1

In these examples all percentages are by weight.

Solution A:  6% of 30% aqueous sodium chlorite solution, water to 100%
Solution B:  4% of 47% aqueous solution of sodium hydroxide, 10% aqueous acetic acid, 3.5% aqueous phosphoric acid (81%), water to 100%

10 x 100g samples were prepared by taking 1g of solution A and 1g of solution B into 98g of water. The solutions were allowed to stand for the time indicated in Table 1 then tested for free chlorous acid by titration with sodium thiosulphate solution. The starting pH of each solution was 3.5.

TABLE 1

| Solution Number | Time (minutes) | Free Chlorous Acid (ppm) |
|---|---|---|
| 1 | 5 | 35.4 |
| 2 | 10 | 50.8 |
| 3 | 15 | 60.8 |
| 4 | 20 | 69.4 |
| 5 | 25 | 69.5 |
| 6 | 30 | 68.9 |
| 7 | 35 | 70.0 |
| 8 | 40 | 68.3 |
| 9 | 60 | 69.5 |
| 10 | 120 | 69.2 |

An experiment was carried out as in Example 1 except that additional acetic acid was added to lower the pH to pH 2, i.e. below the buffered pH value of 3.5.

The total available chlorous acid was determined to be 0.0101%, i.e. 101 ppm.

Example 2

A 100 g sample was prepared as in Example 1. It was allowed to stand and the free chlorous acid concentration in the sample was tested at intervals. The free chlorous acid was removed after each measurement of chlorous acid concentration as a consequence of the titration with sodium thiosulphate solution. The time intervals and the cummulative chlorine dioxide concentration are shown in Table 2.

TABLE 2

| Time (minutes) | Free Chlorous Acid (ppm) |
|---|---|
| 5 | 35.4 |
| 10 | 51.0 |
| 15 | 60.4 |
| 20 | 68.3 |
| 25 | 74.3 |
| 30 | 77.6 |
| 35 | 79.3 |
| 40 | 81.0 |

Comparative Test A

A sample prepared as in Example 1 was acidified with acetic acid to pH 2 and analysed for total available chlorous acid = 0.010%.

Example 3

An experiment was carried out as in Example 2 but with 15 minute intervals between measurements. Table 3 shows the cummulative concentration of chlorine dioxide.

TABLE 3

| Time (minutes) | Free Chlorous Acid (ppm) |
|---|---|
| 30 | 69.2 |
| 45 | 81.0 |
| 60 | 86.4 |
| 75 | 91.1 |
| 90 | 94.7 |
| 105 | 98.0 |
| 120 | 99.6 |

The results for solutions 1 to 10 in Example 1 show that after 30 minutes an equilibrium concentration of 60 ppm chlorous acid is reached. This in spite of there being an available chlorous acid concentration of 101 ppm, as shown in Comparative Test A.

Example 2 shows a similar trend but here, the free chlorous acid is being removed every 5 minutes. However, removal of the free chlorous acid causes the equilibrium to shift to produce more free chlorous acid. The amount released is given by the difference between a value and its preceding value. Continued addition of thiosulphate in Example 2 caused the pH of the sample to gradually rise thereby progressively suppressing the rate of release of chlorous acid. It is believed, therefore, that a mechanism that removes free chlorous acid without affecting pH would give rise to greater incremental concentrations of free chlorous acid.

Example 3 shows a similar trend in chlorous acid release to Example 2. Table 3 also showed that an equilibrium concentration of 69 ppm free chlorous acid exists in solution before titration. In this experiment, continued removal of free chlorous acid at quarter hourly intervals eventually caused the solution to yield 98.61% of its potential available chlorous acid as free chlorous acid. Although this took 120 minutes, the pH was found to increase from its starting value of 4.00 to 5.9 on completion of the test. This was due to the alkalinity of the sodium thiosulphate. This gradual increase in pH during the course of the experiment would result in progressively slower release of free chlorous acid, as found with Example 2.

Example 4

Solution A:    6% of 30% aqueous sodium chlorite, water to 100%
Solution B:    10% glacial acetic acid, 0.60% phosphoric acid (81%), 4% aqueous sodium hydroxide (47% by wt of NaOH), water to 100%

7 x 100 g samples were prepared by taking 1 g of solution A and 1 g of solution B into 98 g of water. The solutions were left to stand for the time indicated in Table 4 then tested for free chlorous acid. The starting pH of each solution was 4.5.

TABLE 4

| Solution Number | Time (minutes) | Free Chlorous Acid (ppm) |
|---|---|---|
| 1 | 5 | 14.5 |
| 2 | 10 | 30.2 |
| 3 | 15 | 33.0 |
| 4 | 20 | 34.0 |
| 5 | 25 | 38.8 |
| 6 | 30 | 55.7 |
| 7 | 60 | 67.6 |

Example 5

An experiment was carried out as in Example 2, with the concentration of free chlorous acid being reduced to zero after each determination as a consequence of titration with sodium thiosulphate solution.

TABLE 5

| Time (minutes) | Free Chlorous Acid (ppm) |
|---|---|
| 5 | 13.5 |
| 10 | 26.2 |
| 15 | 33.8 |
| 20 | 40.6 |
| 25 | 47.4 |
| 30 | 49.9 |
| 60 | 66.8 |

The results for Example 4 show that at pH 4.5, establishing an equilibrium takes longer than at pH 3.5. Comparison of the results obtained for Example 5 and 2 suggests that the alkalinity of the thiosulphate had a marked suppressive affect on the former's rate of release of chlorous acid, probably due to its higher starting pH.

Stability of Activated Solution

Example 6 and Comparative Test B

Solution A:    7% sodium chlorite (30% aqueous solution), water to 100%
Solution B:    12.5% aqueous acetic acid (80%), 4% aqueous sodium hydroxide (47% by wt NaOH), 3.0% phosphoric acid (81%), water to 100%
Solution C:    12.5% acetic acid (80%), 0.84% phosphoric acid (81%), water to 100%

A one kilo sample containing 10 g of solution A, 10 g of solution B and 980 g water was prepared. In a comparative test (Test B) a second sample containing 10 g of solution A and 10 g of solution C was also prepared. Each solution was tested for available chlorous acid at the time intervals shown in Table 6. The buffered solution was also tested for free chlorine dioxide.

6

TABLE 6

| Hours | Example 6 | | Test B |
|---|---|---|---|
| | Chlorous Acid/Chlorine Dioxide | | |
| | Available | Free | Available |
| 0 | 122 | - | 122 |
| 1 | 122 | 54 | 101 |
| 2 | 122 | - | 96 |
| 3 | 121 | - | 85 |
| 4 | 120 | - | 77 |
| 5 | 120 | - | 71 |
| 6 | 120 | - | 65 |
| 7 | 120 | - | 58 |
| 8 | 120 | 53 | 52 |
| 16 | 120 | - | 47 |
| 24 | 120 | 53 | 44 |

The acidified solution (Comparative Test B) became very pale yellow within minutes of being acidified because of the presence of free chlorine dioxide. An hour after preparation, the buffered solution gave a head space chlorine dioxide level of zero whereas the acidified solution gave a value of 10 ppm, which increased to 25 ppm after 24 hours.

A comparison of Example 6 and Comparative Test B shows the effects of complete acidification against the affects of using a buffer. Acidification caused a substantial loss of chlorous acid within the first hour of preparation. This loss continued progressively over the following 24 hours with a 64% reduction in chlorous acid during the test period. In contrast, the buffered product retained its activity over the test period.

The buffered solution was water white and remained so for the duration of the experiment. In contrast, the acidified solution became very pale yellow after several minutes which intensified slightly during the experiment but faded towards the completion of the experiment. These results would suggest that coloured solutions only exist above 50 ppm free chlorine dioxide.

Dilution Equipment

In order to mix and dilute in water the buffer and chlorite concentrated solutions, either a peristaltic pump or a venturi can be used. A pipe leading from each concentrate is connected to an arm of a T-piece. Each of the product arms is also fitted with a non-return valve. To control the dosage rate of each product, both product arms of the T-piece are fitted with either needle valves or orifice plates. The third arm of the T-piece is connected to either a pump or venturi both of which can inject the mixture into a moving stream of water. Both products are to be used at 1% in the activated (diluted) solution.

Conclusions

A safe means of using acidified chlorous acid solutions has been developed which does not release chlorine dioxide into the atmosphere. Solutions so prepared (Activated Solutions) set up an equilibrium concentration of the active species which, if removed by some mechanism is replaced by re-establishment of the equilibrium. Activated solutions have a shelf life of at least several days.

On acidification of sodium chlorite solutions, the reactions that take place are as follows:-

$$10NaClO_2 + 10HCl = 10HClO_2 + 10NaCl$$

$$10HClO_2 = 8ClO_2 + 2HCl + 4H_2O$$

$$8ClO_2 + 4H_2O = 4HClO_2 + 4HClO_3$$

It is believed that by using an acetate buffer, an equilibrium is set up that is,

$$ClO_2^- + CH_3COOH = HClO_2 + CH_3COO^-$$

However, because of the presence of acetate ions in the buffer, the equilibrium lies to the left. This stabilised the activated solution and increase its longevity by minimising the chlorous acid available for further decomposition reactions.

Any mechanism which removes the chlorous acid present causes the equilibrium concentration of chlorous acid to be maintained.

It is to be understood that the above described embodiments of the invention have been described by way of illustration only. Many modifications and variations are possible.

## Claims

1. An aqueous solution comprising a chlorite of an alkali metal or alkaline earth metal and a buffer adapted to maintain the pH of the solution at a value in the range from 3.5 to 6.0, characterised in that the buffer has sufficient acid reserve at the buffered pH to convert substantially all the chlorite to chlorous acid.

2. A solution according to Claim 1 wherein the buffer is adapted to maintain the pH of the solution in the range 3.5 to 5.0.

3. A solution according to any one of the preceding claims wherein the chlorite is sodium chlorite.

4. A solution according to any one of the preceding claims wherein the quantity of chlorite is such as to give 25 to 300 ppm of available chlorous acid.

5. A solution according to Claim 4 wherein the quantity of chlorite is such as to give 50 to 250 ppm of available chlorous acid.

6. A solution according to any one of the preceding claims which comprises 20 to 350 ppm of chlorite.

7. A solution according to any one of the preceding claims wherein the buffer comprises acetic acid and an alkali metal hydroxide.

8. A chlorous acid releasing fluid according to any one of the preceding claims which contains a non-oxidisable surfactant.

9. A solution according to any one of the preceding claims wherein the amount of buffer is in the range 0.1 to 4% by dry weight.

10. A process for producing a chlorous acid releasing solution comprising mixing an aqueous solution of a chlorite of an alkali metal or alkaline earth metal with an aqueous solution of a buffer adapted to maintain the pH of the mixture at a value in the range from 3.5 to 6.0, characterised in that the buffer has sufficient acid reserve to convert substantially all the chlorite to chlorous acid.

11. A process according to Claim 10 wherein the buffer is adopted to maintain the pH at a value in the range from 3.5 to 5.0.

## Patentansprüche

1. Wäßrige Lösung, umfassend ein Chlorit eines Alkalimetalls oder Erdalkalimetalls sowie ein Puffer zum Halten des pH-Wertes der Lösung bei einem Wert im Bereich von 3,5 bis 6,0, dadurch gekennzeichnet, daß der Puffer am gepufferten pH über eine ausreichende Säure-Reserve verfügt, um das im wesentlichen alles Chlorit in chlorige Säure umzuwandeln.

2. Lösung nach Anspruch 1, bei welcher der Puffer den pH-Wert der Lösung im Bereich von 3,5 bis 5,0 hält.

**3.** Lösung nach einem der vorhergehende Ansprüche, bei welcher das Chlorit Natriumchlorit ist.

**4.** Lösung nach einem der vorhergehende Ansprüche, bei welcher die Chlorit-Menge 25 bis 300 ppm verfügbare chlorige Saure liefert.

**5.** Lösung nach Anspruch 4, bei welcher die Chlorit-Menge 50 bis 250 ppm verfügbare chlorige Säure liefert.

**6.** Lösung nach einem der vorhergehende Ansprüche, welche Lösung 20 bis 250 ppm Chlorit aufweist.

**7.** Lösung nach einem der vorhergehende Ansprüche, bei welcher der Puffer Essigsäure und Alkalimetallhydroxid umfaßt.

**8.** Chlorige Säure freisetzendes Fluid nach einem der vorhergehende Ansprüche, die ein nichtoxidierbares Tensid enthält.

**9.** Lösung nach einem der vorhergehende Ansprüche, bei welcher die Puffer-Menge im Bereich von 0,1% bis 4 Prozent Trockenmasse liegt.

**10.** Verfahren zur Herstellung einer chlorige Säure freisetzenden Lösung, umfassend Mischen einer wäßrigen Lösung eines Chlorites eines Alkalimetalls oder Erdalkalimetalls mit einer wäßrigen Lösung eines Puffers zum Halten des pH-Wertes der Mischung bei einem Wert im Bereich von 3,0 bis 6,0, dadurch gekennzeichnet, daß der Puffer über eine ausreichende Säure-Reserve verfügt, um das im wesentlichen alles Chlorit in chlorige Säure umzuwandeln.

**11.** Verfahren nach Anspruch 10, bei welchem der Puffer den pH-Wert bei einem Wert im Bereich von 3,5 bis 5,0 hält.

**Revendications**

**1.** Solution aqueuse comprenant un chlorite d'un métal alcalin ou d'un métal alcalino-terreux et un tampon ajusté pour maintenir le pH de la solution à une valeur dans la plage de 3,5 à 6,0, caractérisée en ce que le tampon a suffisamment de réserve d'acide au pH tamponné pour convertir sensiblement tout le chlorite en acide chloreux.

**2.** Solution selon la revendication 1, dans laquelle le tampon est ajusté pour maintenir le pH de la solution dans la plage de 3,5 à 5,0.

**3.** Solution selon l'une quelconque des revendications précédentes, dans laquelle le chlorite est le chlorite de sodium.

**4.** Solution selon l'une quelconque des revendications précédentes, dans laquelle la quantité de chlorite est telle de façon à donner de 25 à 300 ppm d'acide chloreux disponible.

**5.** Solution selon la revendication 4, dans laquelle la quantité de chlorite est telle de façon à donner de 50 à 250 ppm d'acide chloreux disponible.

**6.** Solution selon l'une quelconque des revendications précédentes qui comprend de 20 à 350 ppm de chlorite.

**7.** Solution selon l'une quelconque des revendications précédentes, dans laquelle le tampon comprend de l'acide acétique et un hydroxyde d'un métal alcalin.

**8.** Fluide libérant de l'acide chloreux selon l'une quelconque des revendications précédentes, qui contient un surfactant non oxydable.

**9.** Solution selon l'une quelconque des revendications précédentes, dans laquelle la quantité de tampon est dans la plage de 0,1 à 4% en poids sec.

**10.** Procédé pour produire une solution libérant de l'acide chloreux comprenant le mélange d'une solution aqueuse d'un chlorite d'un métal alcalin ou d'un métal alcalino-terreux avec une solution aqueuse d'un tampon ajusté pour maintenir le pH du mélange à une valeur dans la plage de 3,5 à 6,0, caractérisé en ce que le tampon a suffisamment de réserve d'acide pour convertir sensiblement tout le chlorite en acide chloreux.

**11.** Procédé selon la revendication 10, dans lequel le tampon est ajusté pour maintenir le pH à une valeur dans la plage de 3,5 à 5,0.